# EUROPEAN PATENT SPECIFICATION

(11) **EP 2 681 565 B1**
(45) Date of publication and mention of the grant of the patent: **28.06.2017**
(21) Application number: 12708381.4
(22) Date of filing: 29.02.2012
(51) Int. Cl.: G01N 33/53, G01N 33/68

(54) **IDENTIFICATION OF SMALL-MOLECULE CANDIDATE THERAPEUTICS CAPABLE OF INHIBITING OR INTERFERING WITH A TARGET PROTEIN-PROTEIN INTERACTION**
IDENTIFIKATION VON KLEINMOLEKULAREN WIRKSTOFFKANDIDATEN ALS HEMMER ODER STÖRER EINER ZIELPROTEIN-PROTEIN-WECHSELWIRKUNG
IDENTIFICATION DE PRODUITS THÉRAPEUTIQUES CANDIDATS À PETITE MOLÉCULE APTES À INHIBER UNE INTERACTION PROTÉINE CIBLE - PROTÉINE OU À INTERFÉRER AVEC UNE INTERACTION PROTÉINE CIBLE - PROTÉINE

(30) Priority: 03.03.2011 GB 201103631
(43) Date of publication of application: 08.01.2014
(73) Proprietor: Oxford University Innovation Limited, Botley Oxford OX2 0JB (GB)
(72) Inventor: RABBITTS, Terence, Oxford, OX3 9DS (GB); TANAKA, Tomoyuki, Leeds Yorkshire LS9 7TF (GB)
(74) Representative: HGF Limited
(86) International application number: PCT/GB2012/050455
(87) International publication number: WO 2012/117245

(56) References cited:
- EP-A2- 0 496 162
- WO-A2-2004/046185
- WO-A2-2005/118887
- WO-A2-2009/045434
- WO-A2-2009/117409
- WO-A2-2010/079345
- US-A1- 2003 129 638
- US-A1- 2008 274 490
- LAFONT VIRGINIE ET AL: "Protein-protein recognition and interaction hot spots in an antigen-antibody complex: Free energy decomposition identifies efficient amino acids", PROTEINS: STRUCTURE, FUNCTION AND GENETICS, JOHN WILEY & SONS, INC, US, vol. 67, no. 2, 1 May 2007 (2007-05-01), pages 418-434, XP002599581, ISSN: 0887-3585, DOI: 10.1002/PROT.21259 [retrieved on 2007-01-26]
- ARKIN M R ET AL: "SMALL-MOLECULE INHIBITORS OF PROTEIN-PROTEIN INTERACTIONS: PROGRESSING TOWARDS THE DREAM", NATURE REVIEWS. DRUG DISCOVERY, NATURE PUBLISHING GROUP, GB, vol. 3, no. 4, 1 April 2004 (2004-04-01), pages 301-317, XP009047669, ISSN: 1474-1784, DOI: 10.1038/NRD1343
- MIGUEL R NUNEZ ET AL: "Thyroid stimulating autoantibody M22 mimics TSH binding to the TSH receptor leucine rich domain: a comparative structural study of protein-protein interactions", JOURNAL OF MOLECULAR ENDOCRINOLOGY, SOCIETY FOR ENDOCRINOLOGY, GB, vol. 42, no. 5-6, 1 May 2009 (2009-05-01), pages 381-395, XP002599582, ISSN: 0952-5041, DOI: 10.1677/JME-08-0152

## Description

The present invention relates to a screening method for identifying small-molecule candidate therapeutics capable of inhibiting or interfering with intracellular protein-protein interactions, especially cancer associated oncogenic protein interactions. The present invention also provides methods and assays for, *inter alia,* rationalized drug design based on identifying small molecular weight protein-protein interaction inhibitor molecules that emulate antibody therapeutics products and uses thereof.

### BACKGROUND

Protein-protein interactions are central to most biological processes and therefore represent a large and important class of targets for human therapeutics. Therapeutic antibodies as a compound class have some excellent properties: they are highly specific for their molecular targets and they tend to be very stable in human serum. On the other hand, antibodies suffer from difficulties in manufacture, high cost of production and lack of oral bioavailability. In addition, antibodies are not cell permeable and antagonism of intracellular protein-protein systems tend to be limited to oligonucleotide-based (e.g. shRNA) therapies, which block the expression of the targeted protein. For these reasons, protein-protein interactions have been of great interest to drug discovery. However, developing small-molecule antagonists or inhibitors has been difficult and it is generally considered to be undrugable. A number of factors can contribute to the challenge of identifying small, organic compounds that inhibit protein-protein interactions. These include the general lack of small-molecule starting points for drug design, the typical flatness of the interface, the difficulty of distinguishing real from artefactual binding, the size and character of typical small-molecule libraries and characterizing the stiochiometry and site of binding. The shape of a typical protein-protein interface adds to the difficulty of drug discovery, approximately 750-1,500 A°² of surface area is buried on each side of the interface and X-ray structures of protein-protein pairs do not usually show small, deep cavities that look like small molecule binding sites. However, it might not be necessary for a small-molecule to cover the entire protein binding surface because the subset of the interface that contributes to high affinity binding , the "hot spot", is often much smaller and these "hot spots" at a protein interface are particularly adept at binding to proteins, peptides and small molecules. Targeting the interfaces between proteins has huge therapeutic potential and it has long been the desire to identify small-molecule inhibitors (SMI) of protein-protein interactions but this has been regarded as problematic accordingly discovering small molecule drugs that disrupt protein-protein interactions is an enormous challenge.

One strategy for cancer treatment is to target specific mutant proteins with drugs (genotype-specific therapy) that interfere with protein interactions by mutant proteins inside cells. Similarly, protein:protein interactions in other diseases such as neurodegenerative diseases and bacterial or viral infections are also highly relevant. A prerequisite of molecular target drug development is to confirm that a critical mutant molecule is a drug target. In the instance of using antibodies or antibody fragments as the therapeutic agent, nanomolar high affinity single domains selected by, for example, using intracellular antibody capture technology (IAC) (Tanaka and Rabbitts Nat Protoc, 2010, 5(1), 67-92) can be used to show that blockade of protein-protein interactions is sufficient to block tumour initiation and to control existing tumours. Subsequently, susceptibility can be demonstrated in preclinical mouse models. This is in effect target validation and provides information about that the binding site of the single domain of the target protein inside cells will have therapeutic value if a chemical compound can be found that emulates the single domain binding region. Most antibody based therapeutics and other uses of antibodies require affinity maturation (e.g. by secondary immunization; phage selection maturation) before they are brought to clinical use. Normally, as is the prevailing wisdom in the art, the effort in antibody development relies on methods to increase the affinity of binding. High affinity or affinity matured antibodies, antibody fragments or single domains are used as the starting point to define a binding site on an antibody as a critical site for effect on disease however, high affinity does not allow diverse chemical library screening as the single domain affinities are too high and the starting affinities of the chemical compounds within mixed libraries are generally low. Furthermore, the surface area with which single domains bind to target proteins can be large since the binding sites has amino-acids from three binding complementary determining regions (CDR).

The RAS oncoproteins are membrane-associated molecular switches that function to transduce extracellular signals to a panoply of intracellular response mechanisms. Activating mutations in *RAS* genes are present in about 15% of all cancers and perhaps as many as 30% of metastatic human cancers. It is known from the prior art that RAS proteins are involved in cellular signal transduction via a diverse set of protein-protein interactions, including effector molecules such as RAF1, phosphoinositide 3-kinase (PI3K) and Ral guanine nucleotide dissociation stimulator (RALGDS). Accordingly, considerable effort has been expended in understanding how RAS proteins work and, by extension, how their transforming activity can be blocked therapeutically.

A screening method that could identify small-molecule candidate therapeutics capable of inhibiting or interfering with intracellular protein-protein interactions, especially cancer associated oncogenic protein interactions would offer immediate benefit to the art. WO2005118887 discloses methods for identifying a HIV antiviral compound. The identified HIV antiviral compound is an inorganic small molecule or a peptide-based vaccine.

### BRIEF SUMMARY OF THE DISCLOSURE

A method of identifying a small-molecule candidate therapeutic capable of inhibiting or interfering with an intracellular target protein-protein interaction, the method comprising: (i) identifying a high affinity intracellular single domain antibody (iDab) that is associated with an intracellular protein-protein interaction, blockade of which has an effect on a disease or has an adverse biological effect; (ii) determining key contact residues involved in the intracellular protein-protein interaction between target protein and the high affinity iDab; (iii) engineering a derivative of the high affinity iDab based on the key contact residues to produce a de-matured affinity for binding to the target and reduced surface area of interaction with the target as compared with the starting variable domain, so as to bring the engineered iDab's binding affinity into a range required for potential competition with a small-molecule candidate therapeutic; (iv) contacting the protein target with the engineered ¡Dab and a small-molecule candidate therapeutic and; (v) determining whether the small-molecule candidate is an emulator of engineered iDab. It will be appreciated that step (i) is target validation and provides information about the binding site of the single domain of the target protein inside cells that will have therapeutic value if a chemical compound can be found that emulates the single domain biding region. In steps (ii) and (iii) the reduction in affinity reduces the binding strength without losing binding specificity to the target by mutation of residues directly contacted to the target (de-maturation). Steps (iv) and (v) provide the interaction steps between the screening binding target and binding partners such as small molecular weight compounds that emulate single variable domain or antibody fragment binding surface. The small molecular weight compounds that emulate single variable domain or antibody fragment binding surface can be selected from a library of small molecular weight candidate therapeutics, a strong binding partner will provide a more promising progenitor of specific drugs or candidate therapeutic than weak binding partners.

According to a second aspect of the invention there is provided a method of screening a small-molecule chemical library using an engineered derivative of a high affinity intracellular single domain antibody (iDab) wherein said engineering is based on key contact residues to produce a de-matured affinity for binding to the target and reduced surface area of interaction with a target protein-protein interaction as compared with the starting high affinity iDab, so as to bring the engineered iDab's binding affinity into a range required for potential competition with a small-molecule chemical and wherein the engineered derivative of the iDab has a binding affinity in the range of mM to µM. Preferably, the de-matured single domain has a binding affinity in the region of 100 to 0.1 uM.

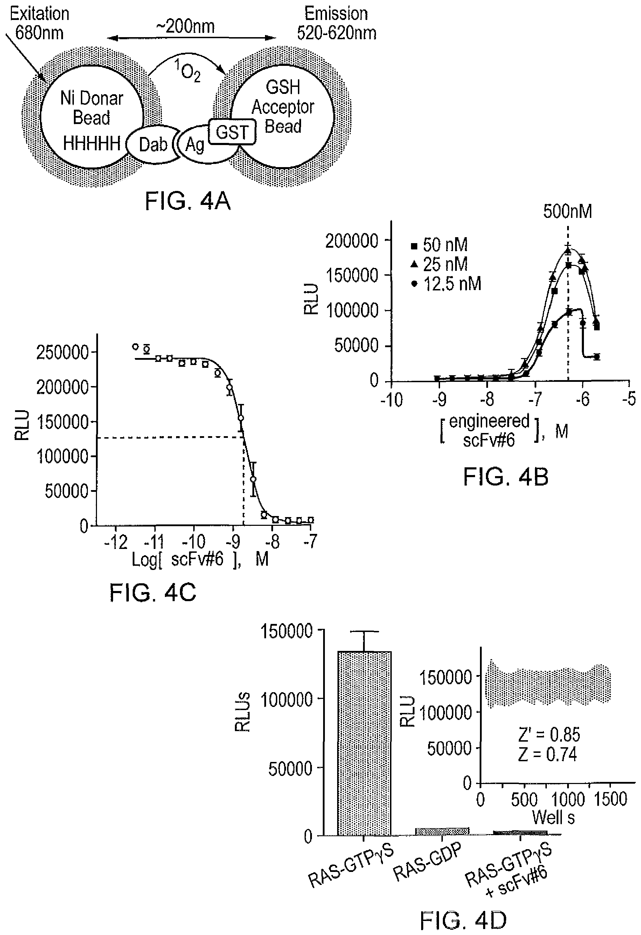

According to a third aspect of the invention there is provided a method such as disclosed above wherein the target protein-protein interaction is an activated RAS-GTP form and the engineered iDab is an anti-RAS single domain and wherein the engineered iDab anti-RAS single domain target carries combined mutation with T28AJS3ONT31A on CDR1 and RIOOG/ F1OIG on CDR3. An example of the binding assay of the third aspect of the invention is shown in Figure 4A however other assays such as cell-based assays in which RAS and VH interaction delivers a signal may be equally applicable. In addition, inhibitors of RAS protein-protein interactions can be assayed in these same assays. The assay of the disclosure provides a strategy for using interaction of RAS with de-matured anti-RAS VH as the primary event for compound identification and subsequent testing of such compounds with RAS-VH and Ras interaction with its natural partner proteins.

### BRIEF DESCRIPTION OF THE DRAWINGS

Embodiments of the invention are further described hereinafter with reference to the accompanying drawings, in which:
Figure 1 shows a schematic representation of antibody proteins and derivative fragment formats.
Figure 2 shows glycine/alanine scanning of anti-RAS VH#6 CDRs using mammalian two hybrid assays.
Figure 3 shows affinity de-maturation of anti-RAS VH#6.
Figure 4 shows the 1536-well AlphaScreen assay.
Figure 5 shows hit compounds inhibiting interaction of de-matured anti-RAS antibody with RAS using Alpha-screen.

### DETAILED DESCRIPTION

Throughout the description and claims of this specification, the words "comprise" and "contain" and variations of them mean "including but not limited to", and they are not intended to (and do not) exclude other moieties, additives, components, integers or steps. Throughout the description and claims of this specification, the singular encompasses the plural unless the context otherwise requires. In particular, where the indefinite article is used, the specification is to be understood as contemplating plurality as well as singularity, unless the context requires otherwise.

Features, integers, characteristics, compounds, chemical moieties or groups described in conjunction with a particular aspect, embodiment or example of the invention are to be understood to be applicable to any other aspect, embodiment or example described herein unless incompatible therewith. All of the features disclosed in this specification (including any accompanying claims, abstract and drawings), and/or all of the steps of any method or process so disclosed, may be combined in any combination, except combinations where at least some of such features and/or steps are mutually exclusive. The invention is not restricted to the details of any foregoing embodiments. The invention extends to any novel one, or any novel combination, of the features disclosed in this specification (including any accompanying claims, abstract and drawings), or to any novel one, or any novel combination, of the steps of any method or process so disclosed.

The reader's attention is directed to all papers and documents which are filed concurrently with or previous to this specification in connection with this application and which are open to public inspection with this specification, and the contents of all such papers and documents are incorporated herein by reference.

Reference herein to "high affinity" is intended to mean that the binding affinity of a ligand to a receptor is in the nanomolar (nM) range and reference to a "de-matured binding affinity" is intended to mean that the binding affinity of a ligand to a receptor is in the millimolar (Mm) to micromolar (µM) range.

Reference herein to "iDab" is intended to refer to an intracellular single domain (designated **i**ntracellular **D**omain **a**nti**b**ody).

Reference herein to "anti-RAS VH#6" is intended to refer to the iDab which binds with high affinity and specificity to the RAS switch I region, which is where the RAS effectors bind.

Results have shown that a single antibody variable region scaffold can be used inside cells to block the function of cancer-associated oncogenic proteins such as mutant RAS and LMO2. In addition these intracellular single domains (iDabs) will block protein-protein interactions in cells to the extent that a biological effect in the form of control of tumorigenesis in a mouse pre-clinical model can be observed. The methods of the present invention provide a convenient way to develop small-molecule and small molecular weight compounds that emulate iDab binding surface and can replace iDab as a lead therapeutic drug. The small molecule antibody technology of the present invention relies primarily on the development of a high affinity single domain for which structural data can be obtained and the subsequent engineering of affinity de-maturation of the iDab binding residues within CDRs prior to screening small-molecules. De-maturation can also be achieved by mutational analysis of the single domain CDRs. De-maturation or reduction in binding affinity is against the usual dogmas of work with antibodies where increased affinity (i.e affinity maturation) is usually considered the desired molecule.

### Plasmids

The triplex vector was used for "one plasmid" mammalian two-hybrid assays (M2H). The plasmid allows expression of a prey protein-VP16 activator domain fusion protein driving from the elongation factor *EF1a* promoter. An anti-RAS VH single domain cDNA sequences was cloned in-frame with the VP16 transactivator domain (AD) controlled by human *EF1a* promoter and a bicistronic mRNA comprising the Gal4 DNA binding domain (DBD)-bait fusion protein-IRES-Renilla luciferase controlled by the SV40 promoter. For glycine/alanine mutation scanning using a mammalian two hybrid assay, RAS cDNA (amino acid 1-166) was sub-cloned in-flame with the Gal4DBD. All constructs were sequenced to confirm in-frame fusion of the inserts with fusion partners.

### Site-directed mutagenesis for glycine/alanine scanning

Mutagenesis of the complementarity determining region (CDR) residues of the anti-RAS VH#6 were performed by PCR assembly method using pEFVP16-VH#6 or subsequent VH#6 CDR mutant triplex constructs as the parental template for the PCR. Each mutagenesis comprised synthesis of two overlapping PCR products using mutant oligonucleotides (step one PCR), followed by complete fragment assembly (step two PCR) and sub-cloning into Sfil / Not I regions of the VP16 AD of the triplex vector. Step one PCR reactions contained 0.5 µM of each reverse mutagenesis oligonucleotide plus 0.5 µM the EFFP2 primer (5'- GGAGGGGTTTTATGCGATGG -3')(SEQ ID NO:1) (a forward primer binding within the *EF-1a* promoter) or 0.5 µM of the forward mutagenesis oligonucleotide plus 0.5 µM of the VP162R primer (5'- CAACATGTCCAGATCGAA -3') (SEQ ID NO:2) (a back primer binding within the VP16 activator domain sequence), 1 U KOD DNA polymerase (TOYOBO), 2 mM MgCl₂, 0.2 mM dNTPs, 1 x KOD PCR buffer and 10 ng pEFVP16-VH#6 template. PCR reactions were carried out by denaturation at 95 °C for 2 min, followed by 40 cycles of 95 °C for 20 sec, 61 °C for 10 sec and 70 °C for 20 sec with final extension at 70 °C for 5 min. The PCR products were electrophoresed on 2% agarose gels, extracted and purified using QIAquick Gel Extractions (Qiagen). Purified PCR fragments were used as templates for step two PCR reactions. Step two PCR reactions used EFFP2 and VP162R as primers and equal amounts of the step one fragments as templates (approximately 1 ng each). Reactions were carried out under the same thermo-cycler conditions as step one PCR. The assembled PCR fragments were purified using QIAquick PCR purification kits (Qiagen), digested with Sfil and Notl, purified by agarose gel electrophoresis and cloned into the Sfil and Not1 sites of the Triplex vector. The final constructs were verified by DNA sequencing.

### Mammalian Two Hybrid (M2H) luciferase assays

Chinese hamster ovary (CHO) cells were grown in DMEM medium with 10 % foetal calf serum, penicillin and streptomycin. A Firefly luciferase reporter CHO cell line called (CHO-Luc15) was used for mammalian two hybrid luciferase assays. The CHO-Luc15 were seeded in 12-well culture plates the day before transfection and grown until more than 90 % confluent. One µg triplex vector was transfected to obtain (co-expression of a mutant form of VH#6-VP16 fusion (prey) with GAL4DBD-HRASG12V (or control) bait and the Renilla luciferase for normalising transfection efficiencies) using 2ul Lipofectamine 2000 according to the Manufacturer's instructions. After 48 hours, the cells were harvested, lysed and assayed using the Dual-Luciferase Reporter Assay System (Promega) according to the Manufacturer's instructions. The data represent a minimum of three experiments for each point and each of which was performed in duplicate. Values are normalised for stimulated Firefly luciferase levels compared with levels for transfected Renilla luciferase.

### Protein expression and purification

For preparation of recombinant GST fusion RAS proteins, pGEX-HRAS(wt) and pGEX-HRAS(G12V) plasmids were transformed into E. coli C41(DE3). Bacterial cells were cultured at 37 °C to an OD₆₀₀ of 0.6 and induced with IPTG (isopropyl 1-thio-beta-D-galactopyranoside, final 0.1 mM) at 30 °C for 5 hours. The bacteria cultures were harvested by centrifugation and the cell pellets were resuspended in 140 mM NaCl, 2.7 mM KCI, 10 mM NaH₂PO₄, 1.8 mM KH₂PO₄, 1 mM EDTA, 2 mM MgCl₂, pH 7.4. The proteins were extracted by cell disruption (Constant Systems Ltd., UK) at 25, 000 psi at 4 °C. The GST fusion proteins were purified by glutathione-sepharose column chromatography (GE Healthcare), eluting with 50 mM Tris-HCl pH8.0, 10 mM reduced glutathione, 1 mM DTT, 2 mM MgCl₂. The eluted proteins were dialysed against 50 mM Tris-HCl pH8.0, 1 mM DTT, 2 mM MgCl₂ and concentrated to 10 mg/ml using a Biomax-30 ULTRAFREE-15 centrifugal filter device (Millipore). To exchange endogenous guanidine nucleotides bound to RAS to GDP or GTP analogue, purified GST-RAS proteins were loaded with GTP□S or GDP (Sigma). The GST-RAS proteins (0.1 mM final) were diluted in Guanidine nucleotide loading buffer (20 mM Tris pH 7.4, 5 mM MgCl₂, 10 mM EDTA, 1 mM DTT, 10 % glycerol) and added GDP or GTP□S (1 mM final). After incubation at 30°C for 30 min, MgCl₂ (50 mM final) was added to stop exchange reactions. The GTP analogue loading proteins were aliquoted into 20 µl each, snap frozen and stored at -70 °C until further experiment.

For purification of anti-RAS scFv, the plasmids pRK-HISTEV-scFv or pRK-HISTEV-VH were prepared by sub-cloning scFv or VH fragment into the pRK-HISTEV vector giving in-frame fusion with a 6x histidine tag and a TEV protease site. The plasmids were transformed into C41 (DE3), cultured at 37 °C to an OD₆₀₀ of 0.6 and induced with IPTG (final 0.5 mM) at 16 °C for 12 hours. The protein was extracted from bacteria pellets with the extraction buffer (25mM Na phosphate, pH7.4, 500 mM NaCl, 20 mM imidazole) using cell disruption at 25, 000 psi at 4 °C and purified using His-Trap Ni-affinity columns (GE Healthcare) employing gradient elution (20-300 mM imidazole). When the his-tag peptide was removed, the protein were treated with TEV protease and dialysed against 20 mM Tris-HCl pH8.0, 300 mM NaCl, 20 mM imidazole at 4°C overnight. The scFv was purified again by passing through a Ni-NTA agarose column (Qiagen) and by gel filtration on a HiLoad Superdex-75 HR column (GE Healthcare) in 20 mM Tris-HCl pH8.0, 250 mM NaCl and concentrated to 10 mg/ml for storage.

### Surface plasmon resonance (SPR) assay

The BIAcore T100 (GE Healthcare) was used to measure the binding kinetics of single chain Fv / single domain VH with RAS protein. Firstly, a polyclonal goat anti-GST antibody (GE Healthcare) was immobilised on a CM5 sensorchip (GE Healthcare) by the amine coupling method. To immobilize the antibody on CM5 chip, the chip was first activated by flowing 100 µl mixture of 0.2 M EDC (N-ethyl-N-(dimethylaminopropyl) carbodiimide hydrocloride) and 0.05 M NHS (N-hydroxysuccinimide) at 10 µl/min flow rate. Then, 100 µg/ml anti-GST antibody in 10 mM Sodium acetate, pH 5.0 was injected at 5 ul/min for 900 sec and immobilized until 15,000 ∼ 25,000 RU. After immobilization the chip immediately inactivated by injecting 1 M ethanolamine, pH 8.5 at 10 µl/min for 600 sec. Then 5 µg/ml recombinant GST, GST-RAS-GTP□S or GST-RAS-GDP proteins were injected for trapping on the chip through the immobilised anti-GST antibody. The binding experiments were performed by injection of purified scFv or VH (1 - 400 nM) in HBS-P buffer (Biacore) containing 10 mM HEPES, pH 8.0, 150 mM NaCl and 0.005 % Surfactant P20 with 1 mM MgCl₂ at 25 °C. The RAS and scFv or VH captured chip surface were regenerated by rinsing with 40 µl 10 mM Glycine-HCl at 20 µl/min flow rate. The kinetics rate constants, Kon and Koff were evaluated using the BIA evaluation 2.0.2 software by manufacture. Kd values were calculated from K_{off} and Kₒₙ rate constants (Kd=k_{off}/kₒₙ).

### AlphaScreen assays

The AlphaScreen assay (Perkin Elmer) was performed in a 25 µl final volume in 1536-well white microtiter plates (Greiner). The reaction buffer contained 25 mM HEPES (pH 7.5), 100 mM NaCl, 5 mM MgCl2, 0.005% (v/v) Tween-20 and 0.1% (w/v) BSA. Purified GST-RAS-GTP□S (25 nM final) and His-tagged engineered anti-RAS scFv#6 comprising VH#6 with T28A/S30A/T31A on CDR1 and R100G/F101G on CDR3, VL121 REF and flexible linker peptide (250 nM final) were incubated with GSH-acceptor beads (10 µg/ml final, Perkin Elmer) and the compounds for 60 min at room temperature (RT). The compounds were added at varying concentrations and nickel chaelate-coated donor beads (10 ug/ml) were added and incubation was continued for an additional 30 min at RT. Exposure of the reaction to direct light was prevented and the emission of light from the acceptor beads was measured in the EnVision plate reader (Perkin Elmer) and analyzed using the EnVision manager software

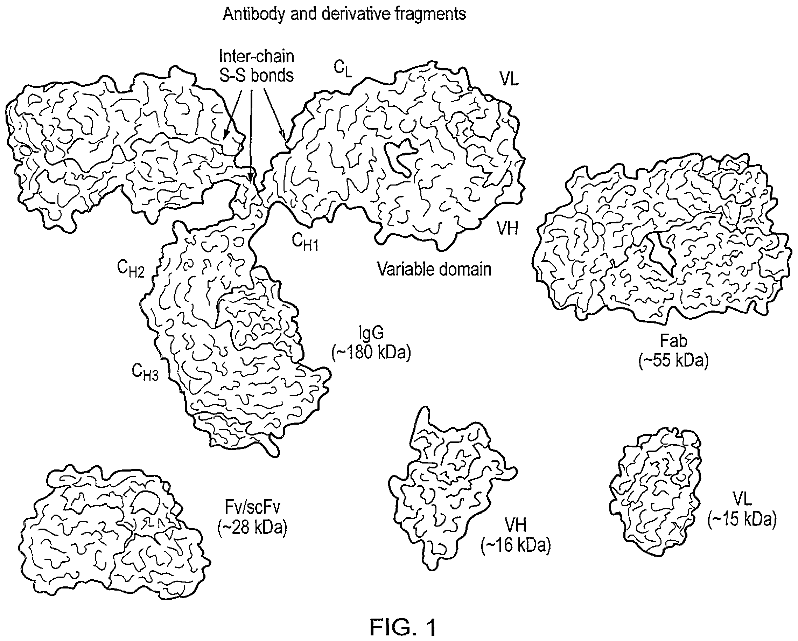

Figure 1 shows surface representations of immunoglobulin and its derivative fragments that can be applicable for extracellular or intracellular use. Whole immunoglobulin G (IgG) comprises two heavy (H, shown in blue and grey) and two light (L, shown in green and light green) chain polypeptides held together by inter-chain disulphide bonds and each chain has a variable (V) and a constant (C) region. The parts of the V-regions that bind to antigen and show most variability are the complementarity determining regions (CDRs, shown in red). The H chain is the longer polypeptide and the H chain C-region is in three domains (CH1, CH2 and CH3) in IgG. The IgG molecule and derivative Fab fragment are shown at the top. Smaller fragments suitable for intracellular use (bottom of the figure) are the Fv which comprises VH and VL held together by VH-VL interaction, single chain Fv (scFv) which comprises VH and VL held by a flexible linker peptide (depicted here by the narrow loop) and single VH or VL domains (right bottom).

Intrabodies are normally single chain Fv fragments comprising variable domains of the immunoglobulin heavy (VH) and light chains (VL). It has been demonstrated that single VH domains have excellent intracellular properties of solubility, stability and expression within the cells of higher organisms and can exhibit specific antigen recognition *in vivo* (Tanaka and Rabbits,2008, J Mol Biol 376, 749-757). Using the intracellular single variable domains (iDabs) were isolated using two distinct antigens and affinities of isolated IDabs ranged between 20 nM and 200 nM.

High affinity single domains are used to define binding sites on an antibody as a critical site for effect on disease however, high affinity (nM) does not allow diverse chemical library screening as the single domain affinities are too high and the starting affinities of the chemical compounds within mixed libraries are generally low. Further the surface area with which single domains bind to target proteins can be large since the binding sites has amino-acids from three binding regions (CDR). Normally, the effort in antibody development relies on methods to increase the affinity of binding. In the methods of the present invention, de-maturation of single domains is employed to reduce the binding affinity and the surface area of the single domain binding sites to bring them into a more compatible range needed for potential competition by chemical compounds (i.e. small-molecule emulators of antibody fragment binding sites). This is achieved after hot-spot analysis defines the critical amino-acids in the three CDRs of the single domain and allows the protein engineering design of a de-matured version of the single domain (the use of single domains is further advantageous because the de-maturation process is simplified by the involvement of only 3 CDRs as opposed to 6 CDR in scFv). Thus this reduction in affinity reduces interaction surface area and affinity by mutation of key residues (de-maturation) but advantageously maintains the specificity by which the single domain binds its target protein.

Screening of small-molecule chemical libraries using de-matured single domains for small-molecule emulators of antibody fragment binding site, in the methods of the present invention, are carried out with de-matured molecules with affinities preferably around 1 uM. As an example, we have de-matured a nM affinity single domain to be around 1uM binding affinity and shown, using ELISA-based *in vitro* assays, that the binding of the uM de-matured single domain is inhibited by the nM single domain.

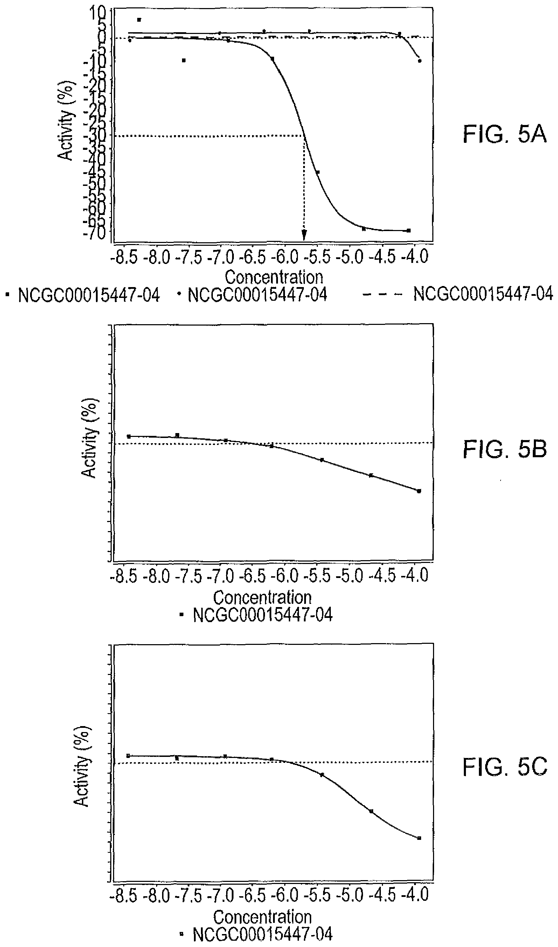

Furthermore, we have used a binding assay involving RAS-GTP (the activated form of RAS) and the 1uM de-matured anti-RAS single domain (in the form of scFv in which the VL is not involved) to exemplify the screening of a small molecule library to select inhibitors of the binding of the single domain to RAS. His-tagged anti-RAS scFv and GST-RAS were bound mixed with beads that can give a signal if scFv and RAS interaction (AlphaScreen). The screen is conducted with His-tagged anti-RAS scFv and GST-RAS plus individual chemical compounds; one was found that was shown to exhibit inhibition kinetics in the range of 2uM (Figure 5A).

These small molecule emulators of antibody fragment binding sites are progenitors of specific drugs.

To our knowledge, the prior art does not teach decreasing affinity in such screening methods since there has been no previous objectives where a less than highest affinity antibody-target interaction is needed. Most antibody uses require affinity maturation (e.g. by secondary immunization; phage selection maturation) before they are brought to clinical use. It is counter-intuitive to de-mature or reduce the binding affinity in this context. For the methods of the of the present invention, the high affinity antibodies will be too high to allow assessment of small-molecule inhibitors; but further and crucially it was totally unexpected that reducing affinity (de-maturation) would result in interaction kinetics that would allows the detection of small-molecule inhibitor hits from library screens. Results have shown surprisingly that using the screening methods of the present invention that a small number of hits were identified that were capable of blocking the protein-protein interaction of RAS and anti-RAS single domain.

### EXAMPLE 1

The methods of the present invention comprise the steps of deriving a high affinity iDab and demonstrating that the iDab interferes with the target protein-protein interaction (PPI). Subsequently, it is necessary to demonstrate in, for example, a biological system such as a transgenic mouse or cell based tumour model that the high affinity iDab blocks or controls a protein-protein interaction resulting in tumourigenesis. Once this is achieved, the crystal structure of antigen-iDab heterodimer is solved so that hot spot analysis of iDab CDRs can be carried out to determine the key residues in the protein-protein interaction. At this stage, iDab derivatives are engineered that have lower binding affinity (determined usually by SPR) whilst maintaining their specificity. The lower affinity or de-matured iDab is then used as the target in a small molecule library screen to identify binding partners or "hits" as candidate therapeutics in for example surface plasmon resonance (SPR) binding to antigen. The crystal structure of antigen small-molecule hit is then solved and the medicinal chemistry of the hit is investigated to identify lead compounds on which further confirmatory tests such as competition assays and cell-based assays can be performed to determine the best compound leads. The methodology can be summarized below:
(i) derive high affinity iDab;
(ii) show iDab interferes with target PPI;
(iii) demonstrate in a biological system that the high affinity iDab will block or control tumourigenesis;
(iv) optionally resolve crystal structure of antigen-iDab heterodimer;
(v) conduct hot spot analysis of iDab CDRs to determine key residues in the interaction;
(vi) engineer iDab derivatives that have lower binding affinity;
(vii) use lower affinity iDab-interacting with target molecule as template for inhibition in small-molecule library screen;
(viii) test hits in SPR binding to antigen;
(ix) resolve crystal structure of antigen-small molecule hits;
(x) medicinal chemistry of hits and repeat (viii) to make lead compounds; and
(xi) carry out further biological testing such as competition assays with leads and cell-based assays.

### EXAMPLE 2

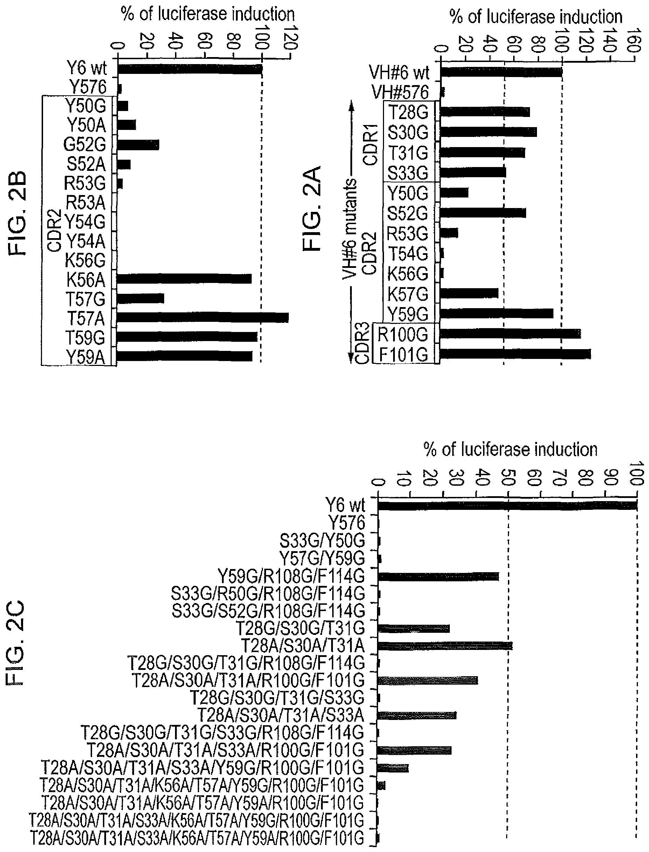

Mammalian two hybrid assays were carried out to delineate key residues for interaction RAS and anti-RAS VH#6 single domain. The triplex plasmids with RAS(G12V) baits (Gal4 DBD fusion) and various CDR mutant preys of anti-RAS VH#6 (VP16 fusions) were transfected into a CHO line with stable integration of the firefly *luciferase* reporter gene (CHOluc15). Interaction signals measured by firefly luciferase gene activation normalized to Renilla luciferase activity (assessed as percentage of luciferase activity compared to positive control using wild type anti-RAS VH#6 (VH#6 wt). Anti-LMO2 VH#576 was used as a negative control. Figure 2A shows VH#6 mutants with glycine substitutions in VH#6 CDR1, 2 and 3. The residue number of mutants on x-axis are according to IMGT numbering system. All respective residues shown here directly contact to RAS protein switch region. Figure 2B shows glycine or alanine mutation on VH#6 and Figure 2C shows combined glycine and/or alanine mutation of VH#6.

### EXAMPLE 3

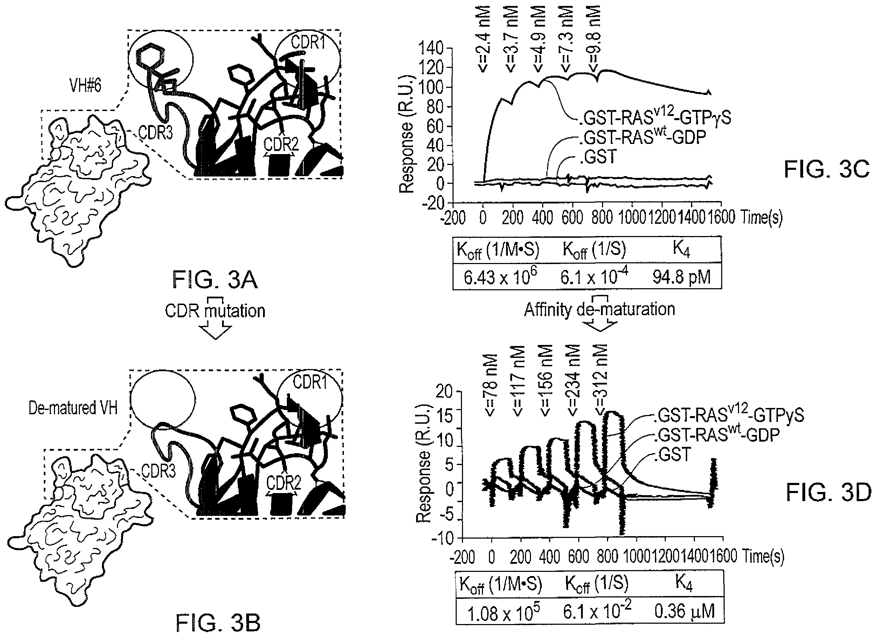

Figure 3 shows affinity de-maturation of anti-RAS VH#6 and the surface representation of the anti-RAS VH#6 wild type (Figure 3A) and engineered VH#6 (carrying combined mutation with T28A/S30A/T31A on CDR1 and R100G/F101G on CDR3) (Figure 3B). CDRs are depicted in red and framework in blue, respectively. Insets are magnification of CDRs regions shown in ribbon form. Amino acid residues involved in the binding to RAS are shown in stick configuration. The mutated residues on engineered VH#6 (Figure 3B) are indicated in green circles. Figures 3C and 3D show affinity measurement of anti RAS VH#6 and engineered VH#6 for RAS-GTP□S using surface plasmon resonance. The binding kinetics were measured using purified scFv format, VH#6 plus VL204 (Figure 3C) or engineered VH#6 plus VLI21 by a single cycle kinetic method on a Biacore T100. The graph shows representative sensorgram of VH#6 (C) and engineered VH#6 (Figure 3D) interact to GST (blue curve), GST-RAS-GDP (green) or GST-RAS-GTP□S (red). The response difference units of the sensorgrams were normalized to the response without capturing GST proteins (on channel Fc1). The inset table in part D shows values for the association (Kₒₙ) (M⁻¹s⁻¹), dissociation rates (K_{off}) (s⁻¹) and the equilibrium dissociation constant (Kd) by determined with the BIAcore T100 evaluation software 2.0.2.

### EXAMPLE 4

Figure 4A shows the principle of the 1536-well AlphaScreen assay which is a bead based non-radioactive amplified luminescent proximity homogenous assay (Perkin Elmer). When a biological interaction like antigen (Ag)-antibody fragment (iDab) protein interaction brings the beads together, a cascade of chemical interactions act to produce greatly amplified signals. On laser excitation at 680nm, a photo-sentitizer in the donor beads coverts ambient oxygen to more excited singlet oxygen which diffused across to reach with thioxene-derivative in the acceptor bead, generating chemiluminescence at 370 nm. This in turn, activates fluorophores that subsequently emit light at 520-620 nm. When an inhibitor, such as a small molecule, interferes with the protein interaction and thus inhibits bead proximity, singlet oxygen produced by the donor cannot reach to acceptor beads, consequently there is no emission light. AlphaLISA GSH Acceptor beads were used to capture GST-tagged RAS loaded with GTPγS. Compounds are added followed by the addition of the His tagged engineered, de-matured scFv#6, comprising engineered VH#6 plus VLI21 fragment that binds to activated RAS with reduced affinity as developed by the mutagenesis evaluation of the VH binding residues (Figures 2 and 3). Following incubation, Ni Donor AlphaScreen beads were added and chemi-luminescence signal detected using a PE Envision plate reader. Figure 4B shows optimization of interacting partners shows a characteristic "hook-effect" above 500nM of the engineered, de-matured scFv#6 and a drop in signal below 25 nM RAS-GTPγS. For the assay, 250nM de-matured scFv#6 and 25 nM RAS-GTPγS using 10 µg/mL acceptor and donor beads were used. Figure 4C shows as control, we observed potent (2nM) inhibition of the de-matured scFV by the high affinity competitor scFv#6, comprising wild type VH#6 plus VL204. Figure 4D shows a histogram of control wells from a 1536-well plate where either the competitor scFv#6 or RAS^{wt} loaded with GDP (inactive form) were used as negative controls. Inset shows a scattergram of the signal obtained in the 1536-well format; Z-factors, 0.74 support a robust assay.

### EXAMPLE 5

Figure 5 shows hit compounds inhibiting interaction of de-matured anti-RAS antibody with RAS using Alpha-screen. The Sigma-Aldrich LOPAC¹²⁸⁰ of 1280 bioactive compounds collection and Tocris compounds library were screened to validate the Alpha-screen assay as HTS. Three compounds showing reduction in the binding of de-matured anti-RAS scFv to GTP-form of RAS were identified.

Figures 5A, 5B and 5C show dose-response inhibition curves of hit compounds, compound A (A), compound B (B) and compound C (C). The graphs show percentage activity for de-matured anti-RAS scFv Alpha interaction (black line and plots), or for irrelevant alpha interaction negtive control assays (blue line and plots), with increasing concentrations of compounds (compared to the activity without compounds). For instance, compound A shows 2 µM IC₅₀.

## Claims

1. A method of identifying a small-molecule candidate therapeutic capable of inhibiting or interfering with an intracellular target protein-protein interaction, the method comprising:
(i) identifying a high affinity intracellular single domain antibody (iDab) that is associated with an intracellular protein-protein interaction, blockade of which has an effect on a disease or has an adverse biological effect;
(ii) determining key contact residues involved in the intracellular protein-protein interaction between target protein and the high affinity iDab;
(iii) engineering a derivative of the high affinity iDab based on the key contact residues to produce a de-matured affinity for binding to the target and reduced surface area of interaction with the target as compared with the starting variable domain, so as to bring the engineered iDab's binding affinity into a range required for potential competition with a small-molecule candidate therapeutic;
(iv) contacting the protein target with the engineered iDab and a small-molecule candidate therapeutic and;
(v) determining whether the small-molecule candidate is an emulator of engineered iDab.

2. A method according to claim 1 wherein the intracellular target protein-protein interaction is critical to the disease or adverse biological effect and is between endogenous proteins or proteins from pathogens.

3. A method according to either claim 1 or 2 wherein the intracellular protein-protein interaction involves a cancer-associated oncogenic protein.

4. A method according to any preceding claim wherein the high affinity iDab has a binding affinity in the nanomolar range.

5. A method according to any preceding claim wherein the engineered derivative of the iDab has a de-matured affinity for the target protein of between 10-100 times less than with the starting iDab.

6. A method according to any preceding claim wherein the high affinity (iDab) is identified by intracellular antibody capture technology or a method suitable for selecting antigen-specific antibody fragments.

7. A method according to any preceding claim wherein the key contact residues of an antigen-antibody complex heterodimer in step (ii) are determined by hot spot analysis of CDRs.

8. A method according to any preceding claim wherein step (iii) comprises mutation of the key contact residues.

9. A method according to claim 8 wherein the mutation is by sequential glycine and/or alanine scanning.

10. A method according to any preceding claim wherein the engineered iDab has a binding affinity in the mM to µM range.

11. A method of screening a small-molecule chemical library using an engineered derivative of a high affinity intracellular single domain antibody (iDab) wherein said engineering is based on key contact residues to produce a de-matured affinity for binding to the target and reduced surface area of interaction with a target protein-protein interaction as compared with the starting high affinity iDab, so as to bring the engineered iDab's binding affinity into a range required for potential competition with a small-molecule chemical and wherein the engineered derivative of the iDab has a binding affinity in the range of mM to µM.

12. A method according to claim 11 wherein the engineered derivative of the iDab has a binding affinity around 0.1 to 100µM.

13. A method according to any preceding claim wherein the target protein-protein interaction is an activated RAS-GTP form and the engineered iDab is an anti-RAS single domain.

14. A binding assay according to claim 13 wherein the engineered iDab anti-RAS single domain target carries combined mutation with T28A/S30A/T31A on CDR1 and R100G/F101G on CDR3

## Patentansprüche

1. Ein Verfahren zur Identifizierung eines niedermolekularen Kandidaten-Therapeutikums, das fähig ist, eine intrazelluläre Ziel-Protein-Protein-Wechselwirkung zu inhibieren oder sie zu beeinträchtigen, das Verfahren umfassend:
(i) Identifizierung eines intrazellulären Einzeldomänen-Antikörpers (iDab) mit hoher Affinität, der mit einer intrazellulären Protein-Protein-Wechselwirkung assoziiert ist, deren Blockade einen Effekt auf eine Krankheit hat oder einen negativen biologischen Effekt hat;
(ii) Bestimmung von Schlüssel-Kontaktresten, die in die intrazelluläre Protein-Protein-Wechselwirkung zwischen Ziel-Protein um dem iDab mit hoher Affinität involviert sind;
(iii) Konstruktion eines Derivats des iDab mit hoher Affinität, auf Grundlage der Schlüssel-Kontraktreste, um eine de-maturierte Affinität für die Bindung an das Ziel und eine reduzierte Wechselwirkungs-Oberfläche mit dem Ziel zu erzeugen, verglichen mit der anfänglichen variablen Domäne, um die Bindungsaffinität des konstruierten iDab in einen Bereich zu bringen, der für potentielle Kompetition mit einem niedermolekularen Kandidaten-Therapeutikum erforderlich ist;
(iv) Kontaktieren des Protein-Ziel mit dem konstruierten iDab und einem niedermolekularen Kandidaten-Therapeutikum und;
(v) Bestimmung, ob der niedermolekulare Kandidat den konstruierten iDab nachahmen kann.

2. Ein Verfahren gemäß Anspruch 1, wobei die intrazelluläre Ziel-Protein-Protein-Wechselwirkung kritisch für die Krankheit oder den negativen biologischen Effekt ist und zwischen endogenen Proteinen oder Proteinen von Pathogenen stattfindet.

3. Ein Verfahren gemäß entweder Anspruch 1 oder Anspruch 2, wobei die intrazelluläre Protein-Protein-Wechselwirkung ein Krebs-assoziiertes onkogenes Protein umfasst.

4. Ein Verfahren gemäß einem vorhergehenden Anspruch, wobei der iDab mit hoher Affinität eine Bindungsaffinität im nanomolaren Bereich hat.

5. Ein Verfahren gemäß einem vorhergehenden Anspruch, wobei das konstruierte Derivat des iDab eine de-maturierte Affinität für das Ziel-Protein von 10 - 100 mal weniger als der anfängliche iDab hat.

6. Ein Verfahren gemäß einem vorhergehenden Anspruchs, wobei der (iDab) mit hoher Affinität mittels *Intracellular Antibody Capture Technology* oder eines Verfahrens, das zur Auswahl antigenspezifischer Antikörperfragmente geeignet ist, identifiziert wird.

7. Ein Verfahren gemäß einem vorhergehenden Anspruch, wobei die Schlüssel-Kontaktreste eines Antigen-Antikörper-Komplex-Heterodimers in Schritt (ii) mittels Hot-Spot-Analyse von CDRs bestimmt werden.

8. Ein Verfahren gemäß einem vorhergehenden Anspruch, wobei Schritt (iii) Mutation der Schlüssel-Kontaktreste umfasst.

9. Das Verfahren von Anspruch 8, wobei die Mutation durch sequentielles Glycin- und/oder Alanin-Scanning erfolgt.

10. Ein Verfahren gemäß einem vorhergehenden Anspruch, wobei der konstruierte iDab eine Bindungsaffinität im mM- bis µM-Bereich hat.

11. Ein Verfahren zum Screening einer Bibliothek von niedermolekularen Chemikalien unter Verwendung eines konstruierten Derivats eines intrazellulären Einzeldomänenantikörpers (iDab) mit hoher Affinität, wobei jene Konstruktion auf Schlüssel-Kontaktresten basiert, um eine de-maturierte Affinität für Bindung an das Ziel und eine reduzierte Wechselwirkungsoberfläche mit einer Ziel-Protein-Protein-Wechselwirkung zu erzeugen, verglichen mit dem anfänglichen iDab mit hoher Affinität, um die Bindungsaffinität des konstruierten iDab in einen Bereich zu bringen, der für potentielle Kompetition mit einer niedermolekularen Chemikalie erforderlich ist, und wobei das konstruierte Derivat das iDab eine Bindungsaffinität im Bereich von mM bis µM hat.

12. Ein Verfahren gemäß Anspruch 11, wobei das konstruierte Derivat des iDab eine Bindungsaffinität um 0,1 bis 100 µM hat.

13. Ein Verfahren gemäß einem vorhergehenden Anspruch, wobei die Ziel-Protein-Protein-Wechselwirkung eine aktivierte RAS-GTP-Form ist und der konstruierte iDab eine anti-RAS-Einzeldomäne ist.

14. Ein Verfahren gemäß Anspruch 13, wobei das konstruierte iDab-anti-RAS-Einzeldomänen-Ziel kombinierte Mutationen mit T28A/S30A/T31A in CDR1 und R100G/F101G in CDR3 trägt.

## Revendications

1. Procédé d'identification d'un agent thérapeutique candidat à petites molécules capable d'inhiber ou d'interférer avec une interaction protéine-protéine cible intracellulaire, le procédé comprenant :
(i) identifier un anticorps à un seul domaine intracellulaire à haute affinité (iDab) qui est associé à une interaction protéine-protéine intracellulaire, dont le blocage a un effet sur une maladie ou a un effet biologique indésirable ;
(ii) déterminer des résidus de contact clés mis en jeu dans l'interaction protéine-protéine intracellulaire entre la protéine cible et l'iDab à haute affinité ;
(iii) mettre au point par génie génétique un dérivé de l'iDab à haute affinité sur la base des résidus de contact clés pour produire une affinité dé-maturée pour la liaison à la cible et une zone de surface d'interaction réduite avec la cible par comparaison avec le domaine variable de départ, de façon à amener l'affinité de liaison de l'iDab mis au point par génie génétique dans une plage requise pour une compétition potentielle avec un agent thérapeutique candidat à petites molécules ;
(iv) mettre en contact la protéine cible avec l'iDab mis au point par génie génétique et un agent thérapeutique candidat à petites molécules et ;
(v) déterminer si le candidat à petites molécules est ou non un émulateur d'iDab mis au point par génie génétique.

2. Procédé selon la revendication 1, dans lequel l'interaction protéine-protéine cible intracellulaire est essentielle pour la maladie ou l'effet biologique indésirable et se situe entre les protéines endogènes ou les protéines provenant de pathogènes.

3. Procédé selon l'une ou l'autre des revendications 1 ou 2, dans lequel l'interaction protéine-protéine intracellulaire met en jeu une protéine oncogénique associée au cancer.

4. Procédé selon l'une quelconque des revendications précédentes, dans lequel l'iDab à haute affinité a une affinité de liaison dans la plage nanomolaire.

5. Procédé selon l'une quelconque des revendications précédentes, dans lequel le dérivé mis au point par génie génétique de l'iDab a une affinité dé-maturée pour la protéine cible d'entre 10-100 fois moins qu'avec l'iDab de départ.

6. Procédé selon l'une quelconque des revendications précédentes, dans lequel l'(iDab) à haute affinité est identifié par une technologie de capture d'anticorps intracellulaire ou une méthode appropriée pour sélectionner des fragments d'anticorps spécifiques d'un antigène.

7. Procédé selon l'une quelconque des revendications précédentes, dans lequel les résidus de contact clé d'un hétérodimère complexe antigène-anticorps à l'étape (ii) sont déterminés par une analyse de points chauds de CDR.

8. Procédé selon l'une quelconque des revendications précédentes, dans lequel l'étape (iii) comprend une mutation des résidus de contact clés.

9. Procédé selon la revendication 8, dans lequel la mutation est par balayage séquentiel de glycine et/ou d'alanine.

10. Procédé selon l'une quelconque des revendications précédentes, dans lequel l'iDab mis au point par génie génétique a une affinité de liaison dans la plage de mM à µM.

11. Procédé de criblage d'une banque chimique à petites molécules à l'aide d'un dérivé mis au point par génie génétique d'un anticorps à domaine unique intracellulaire (iDab) à haute affinité dans lequel ladite mise au point par génie génétique est basée sur des résidu de contact clé pour produire une affinité dé-maturée pour la liaison à la cible et une zone de surface d'interaction réduite avec une interaction protéine-protéine cible par comparaison avec l'iDab à haute affinité de départ, de façon à amener l'affinité de liaison de l'iDab mis au point par génie génétique dans une plage requise pour une compétition potentielle avec un produit chimique à petites molécules et dans lequel le dérivé de génie génétique de l'iDab a une affinité de liaison dans la plage de mM à µM.

12. Procédé selon la revendication 11, dans lequel le dérivé mis au point par génie génétique de l'iDab a une affinité de liaison autour de 0,1 à 100 µM.

13. Procédé selon l'une quelconque des revendications précédentes, dans lequel l'interaction protéine-protéine cible est une forme RAS-GTP activée et l'iDab mis au point par génie génétique est un domaine unique anti-RAS.

14. Procédé selon la revendication 13, dans lequel la cible à domaine unique anti-RAS de l'iDab mis au point par génie génétique porte une mutation combinée avec T28A/S30A/T31A sur CDR1 et R100G/F101G sur CDR3.
